# EUROPEAN PATENT APPLICATION

(11) **EP 2 712 857 A1**
(43) Date of publication of application: **02.04.2014**
(21) Application number: 12185999.5
(22) Date of filing: 26.09.2012
(51) Int. Cl.: C07C 17/02, C07C 17/156, C07C 17/25, C07C 1/24, C07C 11/04, C07C 19/045, C07C 21/06, C08F 14/06

(54) **Process for the manufacture of EDC or VCM starting from ethanol**

(71) Applicant: Solvay SA, 1120 Bruxelles (BE)
(72) Inventor: Degraeve, Paul Julius, 7822 Isières (BE)
(74) Representative: Vande Gucht, Anne

(57) **Abstract**

Process for the manufacture of EDC or VCM starting from ethanol, said process comprising the steps of:
a) subjecting a feedstock comprising ethanol to dehydration, optionally after having been vaporized and/or preheated, to produce an olefins stream containing ethylene;
b) feeding at least part of said olefins stream containing ethylene, optionally after purification, to a direct chlorination reactor and/or to an oxychlorination reactor so as to produce at least one EDC containing stream;
c) optionally purifying this EDC containing stream in heavy and/or light ends;
d) optionally cracking the EDC containing stream so as to produce a VCM containing stream;

said process being characterized by the fact of using waste heat from any of steps b), c) or d) in step a) or vice-versa.

## Description

The present invention relates to a process for the manufacture of 1,2-dichloroethane (EDC) or Vinyl Chloride Monomer (VCM) starting from ethanol (preferably biomass-derived ethanol feedstock).

One of the problems faced with ethylene is that the starting raw materials are from fossil fuels (natural gas, crude oil) which are non-renewable feedstocks.

Applicants however have realized that the production of ethylene and ethylene derivatives compounds would benefit by the replacement of at least a part of the carbonaceous raw materials of fossil origin by renewable resources, such as carbonaceous matter derived from biomass. Of particular interest is the ethanol feedstock which is produced from renewable resources. Such biomass-derived ethanol, also referred to as 'bioethanol' or 'hydrous fuel alcohol' can be prepared in large quantities from biomass via fermentation. The different feedstocks for producing ethanol may be sucrose-containing feedstocks (e.g., sugarcane), starchy materials (e.g., corn, starch, wheat, cassava,... ), lignocellulosic biomass (e.g., switchgrass) and/or agricultural waste.

Processes for dehydration of ethanol to ethylene are well known. These processes typically require temperatures in excess of 300°C where both the olefin and alcohol are in the gas phase and achieve near complete or essentially complete conversion of alcohol to olefin. The thermodynamics favor such high alcohol to olefin conversions only at low pressure, so the process is conventionally operated at or just above atmospheric pressure (e.g., 1 to 2 atm). However, renewable ethanol still contains water, and the presence of water is thermodynamically detrimental to achieving a high conversion in gas-phase ethanol-to-ethylene dehydration.

In a typical gas-phase dehydration process, ethanol is first vaporized and fed to a dehydration reactor (e.g., containing two vessels generally operated in alternating mode to allow regeneration of catalyst) at about 1 atm. Since the dehydration reaction is endothermic, there is an input of heat provided to the reactor. A hot ethylene stream exiting the dehydration reactor is quenched with water and is generally scrubbed with caustic. The ethylene produced must generally meet critical purity specifications. Purification is typically done via cryogenic distillation at elevated pressure, so when ethylene is produced by the gas phase dehydration of ethanol, it must be compressed before purification. For example the quenched and scrubbed ethylene stream is compressed to a pressure from about 0.1 MPa to about 3 MPa, caustic washed, and dried. The dried ethylene stream may be fed to a demethanizer to remove C1 hydrocarbons, and finally to a 'C2' splitter where an ethylene product is produced as an overhead stream, and wherein C3+ are purged. The ethylene produced after purification may be again compressed to the operating pressure of the eventual downstream process.

On the other hand, for producing VCM, two methods are generally employed: the hydrochlorination of acetylene and the dehydrochlorination of ethylene dichloride (1,2-dichloroethane) or EDC. The latter generally happens by thermal cracking and the EDC used therefore is generally obtained by direct chlorination and/or oxychlorination of ethylene.

As namely explained in "Chemical Process Design: Computer-Aided Case Studies", Alexandre C. Dimian and Costin Sorin Bildea, Copyright © 2008 WILEY-VCH Verlag GmbH & Co. KGaA, Weinheim, ISBN: 978-3-527-31403-4, Chapter 7 entitled: "Vinyl Chloride Monomer Process", to date, most of the VCM technologies are based on "balanced" processes.

By this is meant that all intermediates and by-products are recycled in a way that ensures a tight closure of the material balance to only VCM as the final product, starting from ethylene, chlorine and oxygen. The main chemical steps involved are:
1. Direct chlorination of ethylene to 1,2 - ethylene dichloride (EDC):

   C2H4+Cl2→C2H4Cl2+218kJ/mol
2. Thermal cracking (pyrolysis) of EDC to VCM:

   C2H4Cl2→C2H3Cl+HCl-71kJ/mol
3. Recovery of HCl and oxychlorination of ethylene to EDC:

   C2H4+2HCl+0.5O2→C2H4Cl2+H2O+238kJ/mol

Hence, an ideal balanced process can be described by the overall equation:

C2H4+0.5Cl2+0.25O2→C2H3Cl+0.5H2O+192.5kJ/mol

This same document sets forth, namely in sub-chapter 7.7, several ways of saving energy in a "balanced" process as described above, which are all based on using waste heat/energy from the balanced process itself.

The present invention is based on the idea of coupling at least the EDC production part of said VCM production process to the production of ethylene by dehydration of ethanol in order to be able to recover and exchange waste heat between both processes.

To this effect, the present invention relates to a process for the manufacture of EDC or VCM starting from ethanol, said process comprising the steps of:
a) subjecting a feedstock comprising ethanol to dehydration, optionally after having been vaporized and/or preheated, to produce an olefins stream containing ethylene;
b) feeding at least part of said olefins stream containing ethylene, optionally after purification, to a direct chlorination reactor and/or to an oxychlorination reactor so as to produce at least one EDC containing stream;
c) optionally purifying this EDC containing stream in heavy and/or light ends;
d) optionally cracking the EDC containing stream so as to produce a VCM containing stream;
said process being characterized by the fact of using waste heat from any of steps b), c) or d) in step a) or vice-versa.

The process according to the invention preferably uses an impure ethanol feedstock. The impure ethanol feedstock may be anhydrous or hydrous, preferably hydrous. The ethanol feedstock preferably comprises biomass-derived ethanol. The biomass-derived ethanol may be anhydrous or hydrous, preferably hydrous bioethanol. The ethanol feedstock most preferably consists essentially of hydrous bioethanol.

The hydrous bioethanol may have an ethanol content of at least 90%, preferably between 92 and 95%, more preferably between 92.5 and 94%. The hydrous bioethanol may have a water content of from 3 to 8% water, preferably between 5 and 7%. The density of this hydrous bioethanol may be between 807 and 811 kg/m³_{.}

According to the invention, dehydration may employ a vapor-phase or liquid-phase reaction which takes place in a dehydration reactor.

When the dehydration uses a vapor-phase reaction, the ethanol feedstock may be vaporized (for example by the use of steam) and fed to a dehydration reactor.

The dehydration reactor may be operated under isothermal or adiabatic conditions, preferably adiabatic conditions.

The dehydration reactor or reaction zone may contain a fluidized bed or a fixed bed.

Since the dehydration reaction is endothermic, there needs to be an input of heat provided to the reactor. For a single bed adiabatic reactor, the overall endothermic reaction if allowed to go to thermodynamic equilibrium could result in a theoretical temperature drop of 180 °C. Heat management can be addressed by reactor design. Suitable reactor designs include those capable of handling heat fluxes such as fixed bed, fluidized bed, multi-tubular and multiple fixed bed reactors with inter-stage heating zones. Optionally the heat management can be improved by injecting preheated fresh alcohol feed at several points in the reactor bed. The ethanol feedstock may be preheated for example in a furnace to a temperature above the reaction temperature (e.g., at about 400 °C), in order to provide an additional source of heat. A portion of an ethanol recycle stream also can be added at several points along the reactor with additional heating ; alternatively the main portion of such recycle stream may be added to the front end of the reactor. In embodiments when a plurality of dehydration reaction zones operates in series, the gas effluent exiting a reaction zone is heated prior to entering the subsequent reaction zone; such intermediate heating can be done by passing the gas effluent in the same furnace through which the fresh ethanol feedstock is preheated (albeit in separate flow paths).

In a first embodiment of the present invention, at least part of the ethanol is vaporized and/or preheated prior to being fed to the dehydration reactor, by using waste heat from any of steps b), c) or d).

By "preheated" is meant heated without being vaporized.

In a first sub-embodiment, at least part of the ethanol is preheated using waste heat coming from a direct chlorination reactor used in step b).

In a second sub-embodiment, at least part of the ethanol is preheated using waste heat coming from an oxychlorination reactor used in step b);

During step b), at least part of the ethylene comprising stream resulting from dehydration of the ethanol feedstock is transformed (via an exothermic reaction, which may be chlorination and/or oxychlorination) in a EDC containing stream.

Ethylene production from ethanol dehydration is a widely known process. In this process, ethanol is converted into ethylene by means of a catalytic reaction at a temperature of at least 180°C, preferably of at least 300°C.

In preferred embodiments, step (a) comprises contacting the feedstock comprising the biomass-derived ethanol with a dehydration catalyst at a temperature of at least 300°C, more preferably from 310 to 450°C.

In other embodiments, step (a) comprises contacting the feedstock comprising the biomass-derived ethanol with a dehydration catalyst at a temperature of at least 180°C but less than 300°C.

In preferred embodiments, step (a) comprises contacting the feedstock ethanol with a dehydration catalyst at a pressure from 0.5 atm to about 25 atm absolute (from 50 kPa to 2.5 MPa), preferably from 1 to 5 atm (from 101 to 505 kPa), most preferably from 1.1 to 3 atm (from 110 to 303 kPa). Alternate embodiments may comprise a dehydration operating pressure of from 2 to 25 atm (from 202 to 2525 kPa), or from 2 to 20 atm (from 202 to 2020 kPa), or even from 2 to 15 atm (from 202 kPa to 1515 kPa).

The dehydration step (a) is carried out with a dehydration catalyst comprising an acidic component. A large variety of acid catalysts can be used for the dehydration step (a), but the most commonly used solid types include pure or doped high specific surface area gamma-alumina (γ -Al₂O₃), titania/ γ - alumina, magnesium oxide, H-mordenites, silica-aluminate such zeolites (e.g., H-ZSM 5), silica/titania, silicoaluminophosphates ('SAPO' catalysts), and silica-alumina. Other catalysts may be used for liquid-phase dehydration. Preferred examples are solid acid catalysts including isomorphously substituted aluminum phosphate (AlPO) catalysts such as disclosed in WO 2010/085708 (incorporated herein by reference). Unsubstituted AlPO's (see US 3,915,893 incorporated herein by reference) and other strong solid acids such as SAPO's, silicalites, and zeolites (e.g., Zeolite ZSM-5) can be employed. Other nonvolatile acids, such as sulfuric acid, p-toluenesulfonic acid, methanesulfonic acid, heteropoly acids or phosphoric acid may also be used in the dehydration step.

The olefins stream generated in step a) of the process of the invention generally contains ethylene, water, unconverted ethanol and impurities.

This stream is preferably quenched prior to step b) in order to remove water, in an appropriate quench device, generally a column.

The quench device in one embodiment may have a single stage or multiple stages. The multiple-stages quench device of a particular embodiment preferably has two to four stages, more preferably two to three stages. The quench device is optionally in single or multiple housings or towers.

According to a preferred embodiment, the pH of the quench medium is adjusted preferably to greater than 7 as it enters the quench device. Preferably, the pH of the quench medium as it enters the quench device ranges from about 7.1 to about 11.5, more preferably ranges from about 7.5 to about 11, even more preferably ranges from about 8 to about 10 at the time the quench medium enters the quench device. Most preferably, the pH of the quench medium as it enters the quench device is about 9.

The quench device bottoms temperature is from about 180 °F. (82 °C.) to about 300 °F. (149 °C.); preferably from about 180 °F. (82 °C.) to about 250 °F. (121 °C.); more preferably about 200 °F. (93 °C.). The temperature of the quench medium is from about 60 °F. (15 °C.) to about 200 F. (93 °C.); preferably from about 80 F. (27 °C.) to about 140 F. (60 °C.); more preferably about 110 °F. (43 °C.). The quench device may be operated at a pressure that is from about 2 bars (200 kPa) to about 4.5 bars (450 kPa); preferably from about 2 bars (200 kPa) to about 3.77 bars (377 kPa); and more preferably at about 2.4 bars (240 kPa).

The optional step of contacting the olefins stream (exiting the dehydration reactor) with a quench medium removes water from the olefins stream. In one embodiment, the step of contacting the olefins stream with a quench medium removes 95 wt. % or more of the water, preferably 98 wt. % or more, more preferably 99 wt. % or more from the olefins stream based upon the total amount of water in the olefins stream before the olefins stream is quenched. However, in this embodiment of the invention, the quench step does not remove substantially all the water (since a drying (step (b)) is still required). The amount of water remaining after the quench can be up to 0.1 w% (or 1000 wppm) and even up to 0.5 w% (or 5000 wppm).

According to a preferred embodiment of the invention, the olefins stream, eventually quenched, is dried prior to step b), eventually after having been compressed first. Hence, preferably, the olefins stream containing ethylene, eventually quenched, is compressed prior to step b). Also preferably, the olefins stream containing ethylene, eventually quenched and/or compressed, is dried prior to step b).

If the dehydration and/or quench conditions are such that the olefins stream exiting the top of the reactor or the quench column, the case being, is superatmospheric then it must not necessarily be subjected to a compression step.

If it is compressed, the resulting pressure of the compressed olefins stream may be at least 1 bars (100 kPa), or at least 1.1 bars (110 kPa), or at least 2 bars (200 kPa), or at least 3 bars (300 kPa), or at least 4 bars (400 kPa). The resulting pressure of the compressed olefins stream may be 25 bars or less (2500 kPa or less), or 20 bars or less (2000 kPa or less), or at most 15 bars or less (1500 kPa or less).

The gas which exits the compression stage may be advantageously cooled down afterwards by indirect cooling with a cooling media. The cooling media is advantageously chosen among cooling tower water, cold water, atmospheric air and colder gas issued from the process. The cooling media may be preferably chosen among cooling tower water and atmospheric air. The cooling fluid is more preferably cooling tower water.

The compressed olefins stream may be advantageously cooled down under 50°C, preferably under 48°C and more preferably under 45°C but advantageously not under 0°C, preferably not under 5°C and more preferably not under 10°C. At the end of the cool down, some condensates (e.g., water) may be produced. If some condensate is produced, it is preferably separated.

The drying step of the process of an embodiment of the invention may include passing at least part of the olefins stream through a drying unit in order to obtain a dry ethylene-containing stream. By the term "dry" is meant that the ethylene-containing stream contains less than about 100 wppm water, more preferably less than about 10 wppm, and most preferably less than 1 wppm based upon the weight of the olefin product stream.

Any suitable means may be used for drying. The drying step may include (eventually further) compression, fractionation, solid or liquid adsorption, or combinations of two or more of these techniques. A solid adsorption for example may include a molecular sieve adsorption.

In a preferred embodiment, prior to step b), the olefins stream eventually dried and/or submitted to quench and/or compression, is purified by substantially removing all the C3+ therefrom. It is namely so that depending on its use, the ethylene produced by the process described can be further purified as described in the preamble of the application, for instance by being sent to a 'C2/C3' splitter (e.g. a cryogenic distillation unit) where a pure ethylene product is produced as an overhead stream, and wherein C3+ are purged.

In the present invention, the ethylene containing stream produced in step a), optionally purified using the quenching and/or drying and/or compression and/or C3+ purification step described above, is used for making a EDC containing stream by direct chlorination and/or oxychlorination in step b).

The chlorination reaction (usually called direct chlorination) is advantageously carried out in a liquid phase (preferably mainly EDC) containing a dissolved catalyst such as FeCl₃ or another Lewis acid.

In addition, the chlorination reaction is preferably performed in a chlorinated organic liquid medium. More preferably, this chlorinated organic liquid medium, also called liquid stock, mainly consists of EDC.

The chlorination reaction is advantageously performed at temperatures between 30 and 150°C. Good results were obtained regardless of the pressure both at a temperature below the boiling point (chlorination process under subcooled conditions) and at the boiling point itself (process for chlorination at boiling point).

When the chlorination process according to the invention is a chlorination process under subcooled conditions, it gave good results by operating at a temperature which was advantageously greater than or equal to 50°C and preferably greater than or equal to 60°C, but advantageously less than or equal to 80°C and preferably less than or equal to 70°C, and with a pressure in the gaseous phase advantageously greater than or equal to 1 and preferably greater than or equal to 1.1 bar absolute, but advantageously less than or equal to 20, preferably less than or equal to 10 and particularly preferably less than or equal to 6 bar absolute.

A process for chlorination at boiling point may be preferred to usefully recover the heat of reaction. In this case, the reaction advantageously takes place at a temperature greater than or equal to 60°C, preferably greater than or equal to 70°C and particularly preferably greater than or equal to 85°C, but advantageously less than or equal to 150°C and preferably less than or equal to 135°C, and with a pressure in the gaseous phase advantageously greater than or equal to 0.2, preferably greater than or equal to 0.5, particularly preferably greater than or equal to 1.1 and more particularly preferably greater than or equal to 1.3 bar absolute, but advantageously less than or equal to 10 and preferably less than or equal to 6 bar absolute.

The chlorination process may also be a hybrid loop-cooled process for chlorination at boiling point. The expression "hybrid loop-cooled process for chlorination at boiling point" is understood to mean a process in which cooling of the reaction medium is carried out, for example, by means of an exchanger immersed in the reaction medium or by a loop circulating in an exchanger, while producing in the gaseous phase at least the amount of EDC formed. Advantageously, the reaction temperature and pressure are adjusted for the EDC produced to leave in the gaseous phase and for the remainder of the heat from the reaction medium to be removed by means of the exchange surface area.

The molecular chlorine is added in a sufficient amount to convert most of the ethylene and without requiring the addition of an excess of unconverted chlorine. The chlorine/ethylene ratio used is preferably between 1.2 and 0.8 and particularly preferably between 1.05 and 0.95 mol/mol.

The chlorinated products obtained contain mainly EDC and also small amounts of by-products such as 1,1,2-trichloroethane or small amounts of ethane or methane chlorination products.

The oxychlorination reaction is advantageously performed in the presence of a catalyst comprising active elements including copper deposited on an inert support. The inert support is advantageously chosen from alumina, silica gels, mixed oxides, clays and other supports of natural origin. Alumina constitutes a preferred inert support.

Catalysts comprising active elements which are advantageously at least two in number, one of which is copper, are preferred. Among the active elements other than copper, mention may be made of alkali metals, alkaline-earth metals, rare-earth metals and other metals. The catalysts described in Patent Applications EP-A 255 156, EP-A 494 474, EP-A-657 212 and EP-A 657 213, incorporated by reference, are most particularly preferred.

The catalyst may be used in a fixed bed or in a fluidized bed. This second option is preferred. The oxychlorination process is operated under the range of the conditions usually recommended for this reaction. The temperature is advantageously between 150 and 300°C, preferably between 200 and 275°C and most preferably from 215 to 255°C. The pressure is advantageously above atmospheric pressure. Values of between 2 and 10 bar absolute gave good results. The range between 4 and 7 bar absolute is preferred. This pressure may be usefully adjusted in order to attain an optimum residence time in the reactor and to maintain a constant rate of passage for various operating speeds. The usual residence times range from 1 to 60 s and preferably from 10 to 40 s.

The source of oxygen for this oxychlorination may be air, pure oxygen or a mixture thereof, preferably pure oxygen. The latter solution, which allows easy recycling of the unconverted reactants, is preferred.

The reactants may be introduced into the bed by any known device. It can be advantageous to introduce the oxygen separately from the other reactants for safety reasons. These safety reasons also require the gaseous mixture leaving the reactor or recycled thereto to be kept outside the limits of inflammability at the pressures and temperatures in question. It is preferable to maintain a so-called rich mixture, that is to say containing too little oxygen relative to the fuel to ignite.

The hydrogen chloride/oxygen ratio used is advantageously between 3 and 6 mol/mol. The ethylene/hydrogen chloride ratio is advantageously between 0.4 and 0.6 mol/mol.

The chlorinated products obtained contain mainly EDC and also small amounts of by-products such as 1,1,2-trichloroethane.

The EDC separated from the streams of products derived from the chlorination reactor can be mixed or not with the EDC separated from the streams of products derived from the oxychlorination reactor before the EDC cracking step. When both EDC are mixed, they can be mixed totally or partially.

In one embodiment, the EDC containing stream (which according to the above, mainly contains EDC is used to manufacture vinyl chloride monomer (VCM) by cracking.

In the article mentioned in the preamble of the present specification, namely in sub-chapter 7.6, a flow chart (Figure 7.8) showing how the EDC (both "fresh" EDC coming from the (oxy)chlorination and recycled EDC, which has not been cracked and has been separated from the pyrolysis reaction products (VCM and the HCl)) is purified prior to being fed to the pyrolysis reactor. Such a purification is generally carried out in at least 3 steps: first, a purification in "light" impurities (having a boiling point below the one of EDC), generally using a distillation column; then, a purification in "heavy" impurities (having a boiling point above the one of EDC), generally also using a distillation column and finally, on the bottom products of the latter, which still contains some EDC in order to allow advanced removal of impurities, a concentration step, also using a distillation column.

In a third sub-embodiment of the first embodiment of the invention described above, at least part of the ethanol fed to the dehydration reactor is preheated using waste heat available at the top of at least one distillation column used for purifying the EDC containing stream in heavy and/or light ends i.e. in impurities having respectively a boiling point higher or lower than the one of EDC.

And in a fourth sub-embodiment of the first embodiment of the invention described above, at least part of the ethanol fed to the dehydration reactor used in step a) is preheated using waste heat available at the exit of a cracking oven used to crack the EDC containing stream so as to generate a VCM containing stream in step d).

Finally, in a second embodiment of the invention, waste heat from step a) of the process according to the invention is used in any of steps b), c) or d). In a preferred sub-embodiment, waste heat from the dehydration reactor of step a) is used for heating the EDC containing stream prior to cracking it in step d)._

The conditions under which the EDC cracking step may be carried out are known to persons skilled in the art. The EDC cracking can be performed in the presence or in the absence of third compounds among which can be cited the catalysts; the EDC cracking is in this case a catalytic EDC cracking. The EDC cracking is however preferably performed in the absence of third compounds and under the action of heat only ; the EDC cracking is in this case often called pyrolysis.

This pyrolysis (cracking) is advantageously obtained by a reaction in the gaseous phase in a tubular oven. The usual pyrolysis temperatures are between 400 and 600°C with a preference for the range between 480°C and 540°C. The residence time is advantageously between 1 and 60 seconds with a preference for the range from 5 to 25 seconds. The rate of conversion of the EDC is advantageously limited to 45 to 75 % in order to limit the formation of by-products and the fouling of the tubes of the oven.

The separation of the VCM and hydrogen chloride obtained from the stream of products derived from the pyrolysis is carried out according to known modes, using any known device, in order to collect the purified VCM and the hydrogen chloride. Following purification, the unconverted EDC is advantageously conveyed to the pyrolysis oven, after purification

VCM is afterwards preferably polymerized to produce PVC.

The manufacture of PVC may be a mass, solution or aqueous dispersion polymerization process, preferably it is an aqueous dispersion polymerization process.

The expression aqueous dispersion polymerization is understood to mean free radical polymerization in aqueous suspension as well as free radical polymerization in aqueous emulsion and polymerization in aqueous microsuspension.

The expression free radical polymerization in aqueous suspension is understood to mean any free radical polymerization process performed in aqueous medium in the presence of dispersing agents and oil-soluble free radical initiators.

The expression free radical polymerization in aqueous emulsion is understood to mean any free radical polymerization process performed in aqueous medium in the presence of emulsifying agents and water-soluble free radical initiators.

The expression aqueous microsuspension polymerization, also called polymerization in homogenized aqueous dispersion, is understood to mean any free radical polymerization process in which oil-soluble initiators are used and an emulsion of droplets of monomers is prepared by virtue of a powerful mechanical stirring and the presence of emulsifying agents.

The present invention is illustrated in a non limitative way by Figures 1 to 3 attached, which respectively show 3:
- Fig. 1: A flow chart of an industrial process for the dehydration of ethanol in ethylene.
- Fig. 2: A flow chart of an industrial process for the production of VCM from ethylene.
- Fig. 3: A flow chart including different embodiments (processes) of the present invention.

In these figures, identical numbers designate identical or similar process steps, namely:
1: heating
2: dehydration
3: heat recovery
4: quench
5: compression
6: cooling
7: drying
8: purification
9: direct chlorination
10: oxychlorination
11: EDC purification
12: cracking
13: VCM purification

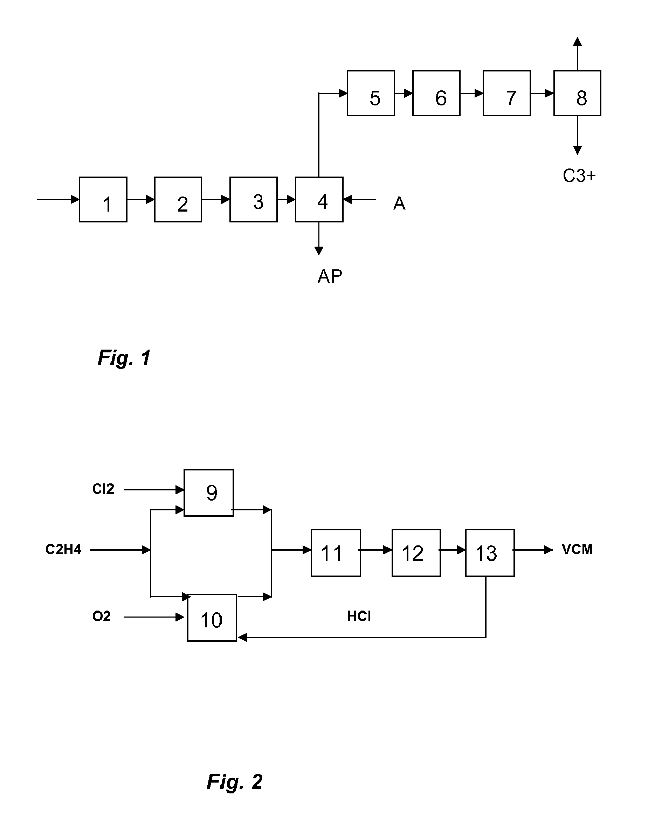

In Figure 1, a liquid ethanol feedstock is heated (for instance in a furnace) in step 1, generating an ethanol vapor which is dehydrated in a reactor (step 2), generating a vapor stream comprising ethylene, water, unconverted ethanol and impurities, among which oxygenates like acetaldehyde.

This vapor stream is first sent to a waste heat boiler in order to recover heat and generate vapor in step 3. It is then sent to a quench tower where it is contacted with an alkaline (A) quench medium (step 4), from which an alkaline liquid (AP) is purged at the bottom. The quenched vapor leaving at the top of this column is first compressed in step 5 then cooled down in step 6 and finally, dried in step 7 before being sent to the final purification step 8, where the C3+ are removed in step 8 at the bottom of a column where substantially pure ethylene exits at the top.

Figure 2 shows a flow chart of a balanced VCM production process starting from ethylene which is fed for the half to a direct chlorination step 9 and for the other half, to an oxychlorination step 10, the former step (9) using the whole of the chlorine and the latter step (10) using oxygen (air) and recycled HCl.

Both steps 9 and 10 generate EDC which is sent to a purification step 11 involving at least one (and preferably at least two) distillation column(s). The purified EDC is sent to a thermal cracking step 12 generating a VCM containing stream which is purified in step 13 also using at least one distillation column, and HCl which is recycled to the oxychlorination step 10.

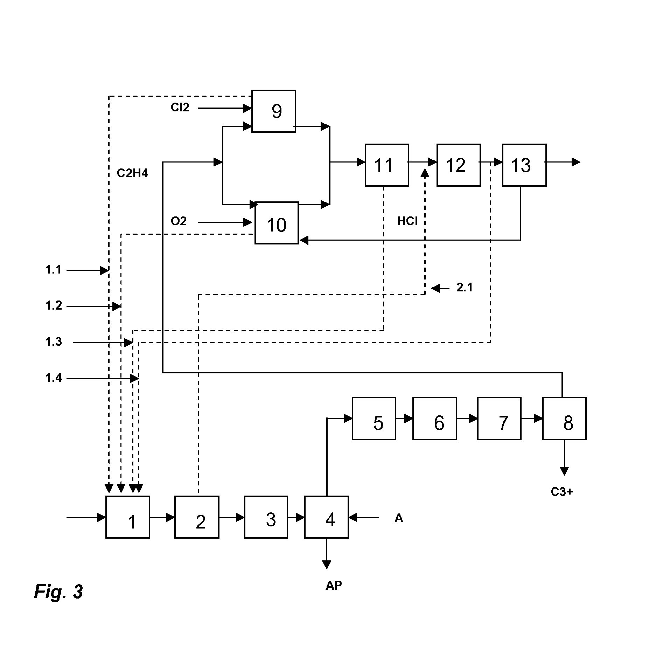

Figure 3 shows a process according to the invention coupling a VCM production process to a process for the production of ethylene by dehydration of ethanol. In this Figure, the "5" embodiments set forth above in the description (actually: the 4 sub-embodiments of the first embodiment, and the one of the second embodiment) are indicated by dotted arrows, namely:
1.1: embodiment where the ethanol is preheated in step 1 using waste heat coming from the direct chlorination step 9
1.2: embodiment where the ethanol is preheated in step 1using waste heat coming from the oxychlorination step 10
1.3: embodiment where the ethanol is preheated in step 1using waste heat available at the top of at least one distillation column used for purifying the EDC in step 11
1.4: embodiment where the ethanol is preheated in step 1using waste heat available at the exit of the cracking step 12
2.1: embodiment where prior to being subjected to cracking in step 12, the EDC containing stream is heated by using the heat of the dehydration reactor from step 2.

Of course, and as shown on Figure 3, these embodiments can be combined.

## Claims

1. Process for the manufacture of EDC or VCM starting from ethanol, said process comprising the steps of:
a) subjecting a feedstock comprising ethanol to dehydration, optionally after having been vaporized and/or preheated, to produce an olefins stream containing ethylene;
b) feeding at least part of said olefins stream containing ethylene, optionally after purification, to a direct chlorination reactor and/or to an oxychlorination reactor so as to produce at least one EDC containing stream;
c) optionally purifying this EDC containing stream in heavy and/or light ends;
d) optionally cracking the EDC containing stream so as to produce a VCM containing stream;
said process being **characterized by** the fact of using waste heat from any of steps b), c) or d) in step a) or vice-versa.

2. Process according to the preceding claim, wherein the ethanol feedstock comprises a biomass-derived ethanol.

3. Process according to any of the preceding claims, wherein prior to being fed to a dehydration reactor used in step a), at least part of the ethanol is vaporized and/or preheated using waste heat from any of steps b), c) or d).

4. Process according to the preceding claim, wherein prior to being fed to the dehydration reactor, at least part of the ethanol is preheated using waste heat coming from a direct chlorination reactor used in step b).

5. Process according to claim 3 or 4, wherein prior to being fed to the dehydration reactor, at least part of the ethanol is preheated using waste heat coming from an oxychlorination reactor used in step b).

6. Process according to any of the preceding claims, wherein the olefins stream containing ethylene is quenched prior to step b).

7. Process according to any of the preceding claims, wherein the olefins stream containing ethylene, eventually quenched, is compressed prior to step b).

8. Process according to any of the preceding claims, wherein the olefins stream containing ethylene, eventually quenched and/or compressed, is dried prior to step b).

9. Process according to any of the preceding claims, wherein the olefins stream containing ethylene, eventually quenched and/or compressed and/or dried, is purified by substantially removing all the C3+ therefrom prior to step b).

10. Process according to any of the preceding claims, wherein at least part of the ethanol fed to a dehydration reactor used in step a) is preheated using waste heat available at the top of at least one distillation column used for purifying the EDC containing stream in step c).

11. Process according to any of the preceding claims, wherein at least part of the ethanol fed to the dehydration reactor used in step a) is preheated using waste heat available at the exit of a cracking oven used to crack the EDC in step d).

12. Process according to any of the preceding claims, wherein waste heat from step a) is used in any of steps b), c) or d).

13. Process according to the preceding claim, wherein waste heat from a dehydration reactor used in step a) is used for heating the EDC containing stream prior to cracking it in step d).

14. Process according to any of the preceding claims, wherein the VCM produced in step d) is polymerized to produce PVC.
